# EUROPÄISCHE PATENTANMELDUNG

(11) **EP 1 468 678 A1**
(43) Veröffentlichungstag der Anmeldung: **20.10.2004**
(21) Anmeldenummer: 04008243.0
(22) Anmeldetag: 05.04.2004
(51) Int. Cl.: A61K 9/12, A61K 31/16

(54) **Pharmazeutisches Schaumaerosol für Dexpanthenol**

(30) Priorität: 08.04.2003 DE 10316280
(71) Anmelder: Aeropharm GmbH, 07407 Rudolstadt (DE)
(72) Erfinder: Neubeck, Walter, 07407 Rudolstadt (DE); Weber, Klaus, 07407 Rudolstadt (DE)
(74) Vertreter: Boeters, Hans Dietrich, Dr.

(57) **Zusammenfassung**

Die Erfindung betrifft ein pharmazeutisches Schaumaerosol (pharmazeutische selbsttreibende Zusammensetzung), umfassend oder bestehend aus
- Dexpanthenol als Wirkstoff,
- Wasser als Wirkstoff-Träger,
- einem oder mehreren Emulgatoren,
- einem oder mehreren lipophilen Komponenten,
- einem oder mehreren fakultativen Hautpflegemitteln,
- einem oder mehreren fakultativen Hilfsmitteln und
einem Treibmittel, bei dem es sich um einen Fluorkohlenwasserstoff oder ein Gemisch aus einem Fluorkohlenwasserstoff und Propan und/oder Butan handelt.

## Beschreibung

Die Erfindung betrifft eine pharmazeutische Aerosols-Formulierung in Form eines Schaums mit sehr guter Schaumqualität für die Verabreichung von Dexpanthenol. Insbesondere wird ein Schaumaerosol für Dexpanthenol beschrieben, das 1,1,1,2-Tetrafluoroethan (= HFA 134a) oder 1,1,1,2,3,3,3-Heptafluoro-n-propan (= HFA 227) als Treibmittel enthält.

Dexpanthenol (Panthenol, D(+)-2,4-Dihydroxy-N-(3-hydroxypropyl)-3,3-dimethylbutyramid) ist der zur Pantothensäure korrespondierende Alkohol, einer Vorstufe des Coenzyms A. Coenzym A spielt eine zentrale Rolle im Stoffwechsel von Säugerzellen und wird vor allem bei geschädigten Zellen, beispielsweise Hautzellen, in großen Mengen umgesetzt. Daher wird Dexpanthenol bevorzugt zur Wundbehandlung verwendet.

Aerosol- Druckgaspackungen, kurz Aerosole genannt, enthalten ein druckverflüssigtes Gas oder Gasgemisch (= Treibmittel) und ein Füllprodukt, welches versprüht werden soll.

Zu den Aerosolen gehören auch die Schaumaerosole, bei denen die Flüssiganteile des Füllprodukts, z.B. eine wäßrige WirkstoffLösung, mit dem druckverflüssigten Treibmittel nicht mischbar sind. Durch Schütteln entsteht in Gegenwart eines Emulgators aus den beiden flüssigen Phasen eine Öl-in-Wasser-Emulsion. Bei deren Austritt aus dem Behälter durch ein Ventil geht die Emulsion wegen des schlagartigen Verdampfens der Gasphase (also der Flüssiggaströpfchen) in einen Schaum über. Durch die Schaumbildung kommt es zu einer raschen, gleichmäßigen und schonenden Auftragung und Verteilung des/der Wirkstoffe. Daher eignen sich Schaumformulierungen besonders für verletzte oder entzündete Haut und auch zum Auftragen in Körperhöhlen.

Schaumaerosole haben gegenüber Lotionen, Cremes oder Salben entscheidende Vorteile. So geben Schaumaerosole ein gutes Hautgefühl, können gut dosiert werden und sind vor Kontaminationen geschützt.

DD 50 625 betrifft ein Verfahren zur Herstellung einer stabilen Emulsion von Panthenol mit Freonen, beispielsweise Difluordichlormethan und Tetrafluordichlorethan.

US 5,679,324 unterscheidet rasch kollabierenden und stabilen Schaum und betrifft einen schnell zerfallenden Schaum mit Panthenol, einem Tensid auf Basis eines Lanolinöl-Derivats, einem Verdickungsmittel, Wasser und einem Fluorkohlenstoff-Gas und/oder Kohlenwasserstoff-Gas wie Isobutan/Propan als Treibmittel.

EP 0 774 957 B1 beschreibt ein Produkt, welches eine aufschäumbare Zusammensetzung aus Wasser, einem schaumbildenden Mittel (Schaumbildner), einem Tensid und einem wasserlöslichen Polymer in einem unter Druck stehenden Behälter sowie - davon räumlich getrennt - ein Treibmittel wie Stickstoff umfaßt. Als schaumbildende Mittel können Propan, Butan, 1,1,1,2-Tetrafluorethan (134 a/P), 1,1,1,2,3,3,3-Heptafluorpropan (HFA 227) oder deren Mischungen verwendet werden. Es wird zwischen prompter und verzögerter Schaumbildung unterschieden, wobei auch Produkte mit Schaumbildner, jedoch ohne Treibmittel beschrieben werden, die zwar prompt Schaum bilden, der jedoch keine Festigkeit besitzen. Ziel ist ein Produkt mit verzögerter Schaumbildung.

In EP 1 014 916 B1 wird ein Herstellungsverfahren für eine Schaum-Hautcreme beschrieben, die eine Fettsäure, einen Moisturiser (z.B. mehrwertigen Alkohol), einen Emulgator und ein Hautpflegeadditiv wie z. B. Dexpanthenol enthält. Die Mischung kann zusammen mit Treibgasen wie Butan und Propan in einen Sprühbehälter abgefüllt werden.

US 2,868,691 beschreibt Aerosolformulierungen zur Inhalation, die einen Wirkstoff, ein halogeniertes kurzkettiges Alkan als Treibmittel und ein Co-solvens enthalten. 1,1,1,2-Tetrafluorethan (HFC-134a) und 1,1,1,2,3,3,3-Heptafluorpropan (HFC-227 ea) sind generisch genannt.

In EP 0 384 371 B1 werden Aerosole, insbesondere Schaumaerosole beschrieben, beispielsweise Rasier- und Duschschäume, die als Treibmittelgemische beispielsweise druckverflüssigtes 1,1,1,2,3,3,3-Heptafluorpropan (HFA 227)/Propan/n-Butan oder HFA 227/Propan/i-Butan enthalten.

In EP 0 779 351 B1 werden azeotrope Mischungen aus 1,1,1,2-Tetrafluorethan (HFC-134a) und 1,1,1,2,3,3,3-Heptafluorpropan (HFC-227 ea) als Treibmittel für Aerosole offenbart, beispielsweise für kosmetische Schäume.

Es ist bekannt, daß die zur Erzeugung eines Schaumes verwendeten Treibmittel einen Einfluß auf die Eigenschaften des Schaums haben. Ein befriedigendes Treibmittel bzw. -gemisch muß bei Raumtemperatur einen Dampfdruck haben, mit welchem im wesentlichen die gesamte Wirkstofflösung ausgetrieben werden kann und gleichzeitig ein Schaum erhalten wird, der fein und langanhaltend ist. EP 1 014 916 B1 spricht beispielsweise die Gefahr des Kollabierens in Abschnitt 0007 ausdrücklich an.

Es hat sich nun gezeigt, daß für ein Schaumaerosol aus Dexpanthenol und den gebräuchlichen Treibmitteln wie Propan/Butan-Gemischen keine optimale Schaumqualität erzielt werden kann. Der so erzeugte Schaum ist nach kurzer Zeit großporig. Zudem verschlechtert sich das Absprühverhalten des Aerosols mit sinkender Füllmenge in der Druckgaspackung.

Die Aufgabe der vorliegenden Erfindung ist die Bereitstellung einer Aerosol- Formulierung für Dexpanthenol mit verbesserter Schaumqualität. Die Aerosol- Formulierung soll einen sich rasch bildenden, feinen, langanhaltenden und stabilen Schaum geringer Dichte bereitstellen. Zudem soll bei sinkender Füllmenge in der Druckgaspackung ein optimales Absprühverhalten gewährleistet sein. Außerdem soll die Aerosol-Formulierung bei Anwendung das Gefühl einer langanhaltenden Kühlung vermitteln.

Gemäß einer Ausführungsform wird die der Erfindung zugrundeliegende Aufgabe gelöst durch ein pharmazeutisches Schaumaerosol (pharmazeutische selbsttreibende Zusammensetzung), umfassend oder bestehend aus
- Dexpanthenol als Wirkstoff,
- Wasser als Wirkstoff-Träger,
- einem oder mehreren Emulgatoren,
- einem oder mehreren lipophilen Komponenten,
- einem oder mehreren fakultativen Hautpflegemitteln,
- einem oder mehreren fakultativen Hilfsmitteln und
- einem Treibmittel, bei dem es sich um einen Fluorkohlenwasserstoff oder ein Gemisch aus einem Fluorkohlenwasserstoff und Propan und/oder Butan handelt.

Das Treibmittel kann aus der folgenden Gruppe ausgewählt sein:
(i) 1,1,1,2-Tetrafluorethan,
(ii) Gemisch aus 1,1,1,2-Tetrafluorethan und Propan,
(iii) Gemisch aus 1,1,1,2-Tetrafluorethan und Butan,
(iv) Gemisch aus 1,1,1,2-Tetrafluorethan, Propan und Butan,
(v) 1,1,1,2,3,3,3-Heptafluor-n-propan,
(vi) Gemisch aus 1,1,1,2,3,3,3-Heptafluor-n-propan und Propan,
(vii) Gemisch aus 1,1,1,2,3,3,3-Heptafluor-n-propan und Butan oder
(viii)Gemisch aus 1,1,1,2,3,3,3-Heptafluor-n-propan, Propan und Butan.

Der Gehalt an Treibmittel kann von 5 bis 20 Gew.-% und vorzugsweise 7 bis 15 Gew.-% betragen, jeweils bezogen auf das Aerosol-Gesamtgewicht.

Das Treibmittel kann aus 1,1,1,2-Tetrafluorethan und Propan/Butan im Massenverhältnis 2 : 98 bis 98 : 2, vorzugsweise 20 : 80 bis 50 : 50 und insbesondere etwa 20 : 80 bestehen.

Ferner kann das Treibmittel aus 1,1,1,2,3,3,3-Heptafluor-n-propan und Propan/Butan im Massenverhältnis 2 : 98 bis 98 : 2, vorzugsweise 20 : 80 bis 50 : 50 und insbesondere etwa 20 : 80 bestehen.

Das erfindungsgemäße Schaumaerosol kann durch n-Butan und/oder i-Butan als Butan gekennzeichnet sein.

Das Massenverhältnis von Propan, n-Butan und i-Butan im Teibmittelgemisch kann etwa 45 % Propan, etwa 52 % n-Butan und etwa 3 % i-Butan betragen.

Ferner kann das erfindungsgemäße Schaumaerosol durch ein Gemisch aus Propan, n-Butan und i-Butan mit einem Dampfdruck von etwa 4,5 bar bei Raumtemperatur gekennzeichnet sein.

Bei dem erfindungsgemäßen Schaumaerosol kann das Treibmittel mit einem Massenverhältnis von 1,1,1,2-Tetrafluorethan, Propan, n-Butan und i-Butan von etwa 20 : etwa 36 : etwa 42 : etwa 2 vorliegen.

Ferner kann bei dem erfindungsgemäßen Schaumaerosol das Treibmittel mit einem Massenverhältnis von 1,1,1,2,3,3,3-Heptafluor-n-propan, Propan, n-Butan und i-Butan von etwa 20 : etwa 36 : etwa 42 : etwa 2 vorliegen.

Der Dexpanthenol-gehalt kann 0,01 bis 10 Gew.-% und vorzugsweise etwa 5 Gew.-% betragen, jeweils bezogen auf das Aerosol-Gesamtgewicht.

Der Wassergehalt kann von 75 bis 90 Gew.-%, insbesondere 80 bis 85 Gew.-% und vorzugsweise etwa 82 Gew.-% betragen, jeweils bezogen auf das Aerosol-Gesamtgewicht.

Der Emulgatorgehalt kann von 0,1 bis 5 Gew.-%, insbesondere 1 bis 3 Gew.-% und vorzugsweise etwa 2 Gew.-% betragen, jeweils bezogen auf das Aerosol-Gesamtgewicht.

Der Gehalt an lipophiler Komponente kann von 0,1 bis 10 Gew.-%, insbesondere 2 bis 6 Gew.-% und vorzugsweise etwa 4 Gew.-% betragen, jeweils bezogen auf das Aerosol-Gesamtgewicht.

Für das erfindungsgemäße Schaumaerosol können ein oder mehrere Emulgatoren aus der folgenden Gruppe vorgesehen sein:
- Cetylstearylalkohol,
- Stearinsäure,
- Stearate, vorzugsweise Triethanolaminstearat, Alkalistearat, Glycerinmonostearat und/oder Ethylenglycolmonostearat,
- Ethoxylate, vorzugsweise Polyethylenglycolate mit Fettalkoholen, insbesondere PEG-Stearat,
- Polyethylenglycole,
- Phosphorsäureester,
- Glucoseester und/oder
- Saccharoseester.

Für das erfindungsgemäße Schaumaerosol können ein oder mehrere lipophile Komponenten aus der folgenden Gruppe vorgesehen sein:
- dünnflüssiges Wachs,
- dünnflüssiges Paraffin,
- Fettsäureester, vorzugsweise Isopropylmyristat, Jojobaöl, Wachsester und/oder Bienenwachsester,
- Milchsäureester,
- Triglyceride, vorzugsweise Rizinusöl, Mandelöl, Sonnenblumenöl, Erdnussöl, Olivenöl und/oder Weizenkeimöl,
- Paraffinöle und/oder
- Silikonöle.

Für das erfindungsgemäße Schaumaerosol kann eine Mischung aus dünnflüssigem Wachs und dünnflüssigem Paraffin als lipophile Komponente vorgesehen sein.

Für das erfindungsgemäße Schaumaerosol können ein oder mehrere Hilfsstoffe aus der folgenden Gruppe vorgesehen sein:
Antioxidantien, Konservierungsstoffe, antimikrobiell wirkende Agenzien, Farbstoffe, Duftstoffe und/oder pH-Regulatoren.

Für das erfindungsgemäße Schaumaerosol können Superoxid-Dismutase, Tocopherol und/oder Ascorbinsäure als Antioxidanz (Hilfsstoff) vorgesehen sein.

Für das erfindungsgemäße Schaumaerosol können Phenoxyethanol, Formaldehyd, Formaldehyd-Lösung, Parabene, Pentandiol und/oder Sorbinsäure als Konservierungsstoff (Hilfsstoff) vorgesehen sein.

Für das erfindungsgemäße Schaumaerosol können ein oder mehrere organischen Säuren, vorzugsweise Peroxyessigsäure und/oder Essigsäure, als pH-Regulator (Hilfsstoff) vorgesehen sein.

Mit dem erfindungsgemäßen Schaumaerosol läßt sich nach dem Auftrag eine unmittelbare Schaumbildung erzielen. so kann die Zeit, die der Schaum zur vollen Entfaltung benötigt 1 bis 15 min betragen, vorzugsweise 2 bis 10 min und insbesondere etwa 3 bis 7 min.

So läßt sich mit dem erfindungsgemäßen Schaumaerosol das Volumen der vollen Schaumentfaltung über die volle Entfaltung hinaus zu mindestens 75 % und vorzugsweise mindestens 90 % etwa 5, vorzugsweise etwa 10 und insbesondere etwa 15 min aufrechterhalten.

Außerdem lassen sich mit dem erfindungsgemäßen Schaumaerosol relative Schaumdichten (im Vergleich zu Wasser) von 0,2 bis 0,001 und insbesondere 0,120 bis 0,050 erreichen.

Mit dem erfindungsgemäßen Schaumaerosol lassen sich durchschnittliche Porengrößen im Bereich von 40 bis 250 und insbesondere 50 bis 200 µm erzielen.

Gemäß einer weiteren Ausführungsform betrifft die Erfindung ein erfindungsgemäßes Schaumaerosol, abgefüllt in einem Druckbehälter, der mit einem Ventil versehen ist.

Gemäß einer weiteren Ausführungsform betrifft die Erfindung ein erfindungsgemäßes Schaumaerosol zur dermalen Applikation, insbesondere für verletzte oder entzündete Haut.

Schließlich betrifft eine Ausführungsform der Erfindung die Verwendung eines erfindungsgemäßen Schaumaerosols zur Applikation in Körperhöhlen.

Überraschenderweise wurde also erfindungsgemäß gefunden, daß durch Verwendung von 1,1,1,2-Tetrafluoroethan (HFA 134a) oder 1,1,1,2,3,3,3-Heptafluoro-n-propan (HFA 227) allein oder im Gemisch mit Propan und/oder Butan als Treibmittel, ein Aerosol für Dexpanthenol mit hervorragender Schaumqualität erhalten werden kann.

Die der Erfindung zugrundeliegende Aufgabe wird unter anderem durch einen teilweisen oder vollständigen Ersatz der üblicherweise verwendeten Treibmittelgemische aus Propan und/oder Butan durch 1,1,1,2-Tetrafluoroethan oder 1,1,1,2,3,3,3-Heptafluoro-n-propan gelöst.

Weiterhin wird mit der Erfindung ein Aerosolprodukt vorgeschlagen, welches aus einer einen beständigen Schaum ergebenden Mischung einer wäßrigen Dexpanthenol-Lösung mit einem Treibmittel der oben beschriebenen Art in einem durch ein Ventil zu betätigenden Druckbehälter, besteht.

Besonders geeignete Treibmittel bzw. Treibmittelmischungen für das erfindungsgemäße, wasserbasierende Aerosol sind:
- 1,1,1,2-Tetrafluoroethan (HFA 134a).
- 1,1,1,2,3,3,3-Heptafluoro-n-propan (HFA 227).
- Ein Gemisch aus HFA 134a und Propan/Butan im Massenverhältnis 2 : 98 bis 98 : 2, vorzugsweise von 20 : 80 bis 50 : 50.
- Ein Gemisch aus HFA 227 und Propan/Butan im Massenverhältnis 2 : 98 bis 98 : 2, vorzugsweise von 20 : 80 bis 50 : 50.

Die Propan/Butan-Mischung kann Propan, n-Butan und/oder i-Butan enthalten. Bevorzugt wird eine Mischung aus 45 % Propan, 52 % n-Butan und 3 % i-Butan verwendet. Die Mischung aus Propan, n-Butan und i-Butan kann bei Raumtemperatur einen Dampfdruck von etwa 4,5 bar aufweisen.

Ein Gemisch aus HFA 134 a, Propan und Butan enthält bevorzugt ca. 20 % HFA 134 a, ca. 36 % Propan, ca. 42 % n-Butan und ca. 2 % i-Butan.

Ein Gemisch aus HFA 227, Propan und Butan enthält bevorzugt ca. 20 % HFA 227, ca. 36 % Propan, ca. 42 % n- Butan und ca. 2 % i- Butan.

Als Treibmittel für Aerosole sind HFA 134a und HFA 227 besonders geeignet, da sie eine geringe Toxizität und einen geeigneten Dampfdruck aufweisen sowie im Gegensatz zu Fluorchlorkohlenwasserstoffen die Ozonschicht nicht schädigen.

Der Treibmittelgehalt der erfindungsgemäßen Aerosol Formulierung beträgt 5 bis 20 Gew.% bezogen auf das Gesamtgewicht des Aerosols. Bevorzugt wird ein Treibmittelgehalt von 7 bis 15 Gew.%.

Der Gehalt an Dexpanthenol der erfindungsgemäßen Aerosol Formulierung beträgt 0.01 bis 10 Gew.% bezogen auf das Gesamtgewicht des Aerosols. Bevorzugt wird ein Wirkstoffgehalt von 5 Gew.%.

Zu den Flüssiganteilen des erfindungsgemäßen Aerosol Füllprodukts gehören die wäßrige Dexpanthenol-Lösung, ein oder mehrere Emulgatoren sowie eine oder mehrere lipophile Komponenten.

Als Emulgatoren eignen sich beispielsweise:
- Cetylstearylalkohol,
- Sterarinsäure und/oder Stearate, beispielsweise Triethanolaminstearat, Alkalistearat, Glycerinmonostearat, Ethylenglycolmonostearat,
- Ethoxylate, wie Polyethylenglycolate mit Fettalkoholen, z. B. PEG-Stearat,
- Polyethylenglycole,
- Phosphorsäureester,
- Glucoseester und/oder
- Saccharoseester.

Es können auch Mischungen aus mehreren Emulgatoren eingesetzt werden. Bevorzugt wird als Emulgator Cetylstearylalkohol eingesetzt.

Geeignete lipophile Komponenten sind beispielsweise:
- dünnflüssiges Wachs,
- dünnflüssiges Paraffin,
- Fettsäureester wie Isopropylmyristat, Jojobaöl, Wachsester, Bienenwachsester,
- Milchsäureester,
- Triglyceride wie Rizinusöl, Mandelöl, Sonnenblumenöl, Erdnussöl, Olivenöl, Weizenkeimöl
- Paraffinöle und/oder Silikonöle
Es können auch Mischungen aus mehreren lipophilen Komponenten eingesetzt werden. Bevorzugt wird eine Mischung aus dünnflüssigem Wachs und dünnflüssigem Paraffin eingesetzt.

Das erfindungsgemäße Füllprodukt kann weitere Hautpflegemittel und/oder Hilfsstoffe, die bekanntermaßen für pharmazeutische Schäume eingesetzt werden, enthalten. Als Hilfsstoffe dienen z. B. Antioxidantien, Konservierungsstoffe, antimikrobiell wirkende Agenzien, Farbstoffe, Duftstoffe und/oder Mittel zur pH-Einstellung.

Als Hautpflegemitteln können beispielsweise Aloe Vera, Jojobaöl, Propolis (Bienenharz), Kollagen, Elastin, Phytosterole sowie Vitamine, z. B. Retinol (Vitamin A), beta-Carotin (Provitamin A), Pyridoxin (Vitamin B6), Ascorbinsäure (Vitamin C), Tocopherol (Vitamin E), essentielle Fettsäuren (wie Vitamin F) und/oder Biotin (Vitamin H) verwendet werden.

Als Antioxidatien eignen sich beispielsweise Superoxid-Dismutase, Tocopherol und/oder Ascorbinsäure.

Als Konservierungsmittel eignen sich beispielsweise Phenoxyethanol, Formaldeydlösung, Parabene, Pentandiol und/oder Sorbinsäure.

Als Farbstoffe können die für pharmazeutische Zwecke zugelassenen Substanzen verwendet werden.

Als Mittel zur pH-Einstellung eignen sich organische Säuren wie Peroxyessigsäure oder Essigsäure. Bevorzugt wird Peroxyessigsäure eingesetzt.

Zur Herstellung des erfindungsgemäßen Schaumaerosols wird zunächst eine Lösung von Dexpanthenol in Wasser hergestellt. Die Dexpanthenol-Lösung wird mit einem oder mehreren Emulgatoren, lipophilen Komponenten und ggf. Hautpflegemitteln und/oder Hilfsstoffen zu einer homogenen Mischung verarbeitet. Diese Mischung wird in einen Aerosolbehälter gefüllt und mit einem Sprühventil verschlossen. Anschließend wird der Aerosolbehälter unter Druck mit dem Treibmittel bzw. Treibmittelgemisch befüllt.

Die erfindungsgemäße pharmazeutische Aerosol- Formulierung ist beispielsweise für eine dermale Verabreichung geeignet.

Die Erfindung wird durch nachstehende Beispiele näher erläutert, ohne aber den Erfindungsumfang damit einzuschränken.

### Beispiel 1:

Die folgenden Stoffe werden zur Herstellung eines erfindungsgemäßen Dexpanthenol Sprays verwendet.

| **Bestandteile** | **Gewicht (g/Spraydose)** |
|---|---|
| Dexpanthenol | 4,63 |
| Cetylstearylalkohol | 1,85 |
| Dünnflüssiges Wachs | 2,78 |
| Dünnflüssiges Paraffin | 1,39 |
| Gereinigtes Wasser | 81,83 |
| Peroxyessigsäure | 0,11 |
| Propan/Butan | 5,93 |
| HFA 134a | 1,48 |
| Gesamt | 100 |

Es wird eine Lösung von Dexpanthenol in Wasser hergestellt. Cetylstearylalkohol, Wachs, Paraffin und Peroxyessigsäure werden gewogen und mit der wäßrigen Dexpanthenol-Lösung zu einer homogenen Öl-in-Wasser Emulsion verarbeitet. Die so erhaltene 5%-ige Dexpanthenol-Emulsion wird in eine Aerosoldose gegeben und mit einem handelsüblichen Ventil verschlossen. Nach Zusatz des Treibmittelgemisches aus Propan, Butan und HFA 134a wird ein entsprechender Sprühkopf aufgesetzt. Bei Niederdrücken des Sprühkopfes entsteht ein feinporiger, stabiler Schaum.

### Beispiel 2:

Zur Herstellung eines Dexpanthenol Sprays werden folgende Stoffe verwendet.

| **Bestandteile** | **Gewicht (g/Spraydose)** |
|---|---|
| Dexpanthenol | 4,63 |
| Cetylstearylalkohol | 1,85 |
| Dünnflüssiges Wachs | 2,78 |
| Dünnflüssiges Paraffin | 1,39 |
| Gereinigtes Wasser | 81,83 |
| Peroxyessigsäure | 0,11 |
| Propan/Butan | 3.71 |
| HFA 227 | 3.70 |
| Gesamt | 100 |

Die Herstellung des Dexpanthenol Sprays erfolgt wie in Beispiel 1.

Die Abfüllung zeigte eine sehr gute Sprühcharakteristik.

### Vergleichsbeispiel 1:

Zur Herstellung eines Dexpanthenol Sprays mit Propan/Butan als Treibmittel werden folgende Stoffe verwendet.

| **Bestandteile** | **Gewicht (g/Spraydose)** |
|---|---|
| Dexpanthenol | 4,63 |
| Cetylstearylalkohol | 1,85 |
| Dünnflüssiges Wachs | 2,78 |
| Dünnflüssiges Paraffin | 1,39 |
| Gereinigtes Wasser | 81,83 |
| Peroxyessigsäure | 0,11 |
| Propan/Butan | 7,41 |
| Gesamt | 100 |

Die Herstellung des Dexpanthenol Sprays erfolgt wie in Beispiel 1.

Die Abfüllung zeigte eine ungenügende Sprühcharakteristik. Der Schaum ist nach kurzer Zeit großporig. Das Absprühverhalten des Aerosols verschlechtert sich mit sinkender Inhaltsmenge der Aerosol Druckgaspackung.

## Patentansprüche

1. Pharmazeutisches Schaumaerosol (pharmazeutische selbsttreibende Zusammensetzung), umfassend oder bestehend aus
- Dexpanthenol als Wirkstoff,
- Wasser als Wirkstoff-Träger,
- einem oder mehreren Emulgatoren,
- einem oder mehreren lipophilen Komponenten,
- einem oder mehreren fakultativen Hautpflegemitteln,
- einem oder mehreren fakultativen Hilfsmitteln und
- einem Treibmittel, bei dem es sich um einen Fluorkohlenwasserstoff oder ein Gemisch aus einem Fluorkohlenwasserstoff und Propan und/oder Butan handelt.

2. Schaumaerosol nach Anspruch 1 mit einem Treibmittel aus der folgenden Gruppe:
(i) 1,1,1,2-Tetrafluorethan,
(ii) Gemisch aus 1,1,1,2-Tetrafluorethan und Propan, (iii)Gemisch aus 1,1,1,2-Tetrafluorethan und Butan,
(iv) Gemisch aus 1,1,1,2-Tetrafluorethan, Propan und Butan,
(v) 1,1,1,2,3,3,3-Heptafluor-n-propan,
(vi) Gemisch aus 1,1,1,2,3,3,3-Heptafluor-n-propan und Propan,
(vii)Gemisch aus 1,1,1,2,3,3,3-Heptafluor-n-propan und Butan oder
(viii)Gemisch aus 1,1,1,2,3,3,3-Heptafluor-n-propan, Propan und Butan.

3. Schaumaerosol nach Anspruch 1 und/oder 2 mit einem Gehalt an Treibmittel von 5 bis 20 Gew.-% und vorzugsweise 7 bis 15 Gew.-%, jeweils bezogen auf das Aerosol-Gesamtgewicht.

4. Schaumaerosol nach mindestens einem der vorhergehenden Ansprüche mit einem Treibmittel aus 1,1,1,2-Tetrafluorethan und Propan/Butan im Massenverhältnis 2 : 98 bis 98 : 2, vorzugsweise 20 : 80 bis 50 : 50 und insbesondere etwa 20 : 80.

5. Schaumaerosol nach Anspruch 1 und/oder 2 und/oder 3 mit einem Treibmittel aus 1,1,1,2,3,3,3-Heptafluor-n-propan und Propan/Butan im Massenverhältnis 2 : 98 bis 98 : 2, vorzugsweise 20 : 80 bis 50 : 50 und insbesondere etwa 20 : 80.

6. Schaumaerosol nach mindestens einem der vorhergehenden Ansprüche mit n-Butan und/oder i-Butan als Butan.

7. Schaumaerosol nach mindestens einem der vorhergehenden Ansprüche mit einem Massenverhältnis von Propan, n-Butan und i-Butan im Teibmittelgemisch von etwa 45 % Propan, etwa 52 % n-Butan und etwa 3 % i-Butan.

8. Schaumaerosol nach Anspruch 6 und/oder 7 mit einem Gemisch aus Propan, n-Butan und i-Butan mit einem Dampfdruck von etwa 4,5 bar bei Raumtemperatur.

9. Schaumaerosol nach mindestens einem der vorhergehenden Ansprüche mit einem Treibmittel mit einem Massenverhältnis von 1,1,1,2-Tetrafluorethan, Propan, n-Butan und i-Butan von etwa 20 : etwa 36 : etwa 42 : etwa 2.

10. Schaumaerosol nach mindestens einem der vorhergehenden Ansprüche mit einem Treibmittel mit einem Massenverhältnis von 1,1,1,2,3,3,3-Heptafluor-n-propan, Propan, n-Butan und i-Butan von etwa 20 : etwa 36 : etwa 42 : etwa 2.

11. Schaumaerosol nach mindestens einem der vorhergehenden Ansprüche mit einem Dexpanthenol-gehalt von 0,01 bis 10 Gew.-% und vorzugsweise etwa 5 Gew. -%, jeweils bezogen auf das Aerosol-Gesamtgewicht.

12. Schaumaerosol nach mindestens einem der vorhergehenden Ansprüche mit einem Wassergehalt von 75 bis 90 Gew.-%, insbesondere 80 bis 85 Gew.-% und vorzugsweise etwa 82 Gew.-%, jeweils bezogen auf das Aerosol-Gesamtgewicht.

13. Schaumaerosol nach mindestens einem der vorhergehenden Ansprüche mit einem Emulgatorgehalt von 0,1 bis 5 Gew.-%, insbesondere 1 bis 3 Gew.-% und vorzugsweise etwa 2 Gew.-%, jeweils bezogen auf das Aerosol-Gesamtgewicht.

14. Schaumaerosol nach mindestens einem der vorhergehenden Ansprüche mit einem Gehalt an lipophiler Komponente von 0,1 bis 10 Gew.-%, insbesondere 2 bis 6 Gew.-% und vorzugsweise etwa 4 Gew.-%, jeweils bezogen auf das Aerosol-Gesamtgewicht.

15. Schaumaerosol nach mindestens einem der vorhergehenden Ansprüche mit einem oder mehreren Emulgatoren aus der folgenden Gruppe:
- Cetylstearylalkohol,
- Stearinsäure,
- Stearate, vorzugsweise Triethanolaminstearat, Alkalistearat, Glycerinmonostearat und/oder Ethylenglycolmonostearat,
- Ethoxylate, vorzugsweise Polyethylenglycolate mit Fettalkoholen, insbesondere PEG-Stearat,
- Polyethylenglycole,
- Phosphorsäureester,
- Glucoseester und/oder
- Saccharoseester.

16. Schaumaerosol nach mindestens einem der vorhergehenden Ansprüche mit einer oder mehreren lipophilen Komponenten aus der folgenden Gruppe:
- dünnflüssiges Wachs,
- dünnflüssiges Paraffin,
- Fettsäureester, vorzugsweise Isopropylmyristat, Jojobaöl, Wachsester und/oder Bienenwachsester,
- Milchsäureester,
- Triglyceride, vorzugsweise Rizinusöl, Mandelöl, Sonnenblumenöl, Erdnussöl, Olivenöl und/oder Weizenkeimöl,
- Paraffinöle und/oder
- Silikonöle.

17. Schaumaerosol nach Anspruch 16 mit einer Mischung aus dünnflüssigem Wachs und dünnflüssigem Paraffin als lipophile Komponente.

18. Schaumaerosol nach mindestens einem der vorhergehenden Ansprüche mit einem oder mehreren Hilfsstoffen aus der folgenden Gruppe: Antioxidantien, Konservierungsstoffe, antimikrobiell wirkende Agenzien, Farbstoffe, Duftstoffe und/oder pH-Regulatoren.

19. Schaumaerosol nach mindestens einem der vorhergehenden Ansprüche und insbesondere nach Anspruch 18 mit Superoxid-Dismutase, Tocopherol und/oder Ascorbinsäure als Antioxidanz (Hilfsstoff).

20. Schaumaerosol nach mindestens einem der vorhergehenden Ansprüche und insbesondere nach Anspruch 18 mit Phenoxyethanol, Formaldehyd, Formaldehyd-Lösung, Parabene, Pentandiol und/oder Sorbinsäure als Konservierungsstoff (Hilfsstoff).

21. Schaumaerosol nach mindestens einem der vorhergehenden Ansprüche und insbesondere nach Anspruch 18 mit einer oder mehreren organischen Säuren, vorzugsweise Peroxyessigsäure und/oder Essigsäure, als pH-Regulator (Hilfsstoff).

22. Schaumaerosol nach mindestens einem der vorhergehenden Ansprüche, abgefüllt in einem Druckbehälter, der mit einem Ventil versehen ist.

23. Verwendung eines Schaumaerosols nach mindestens einem der vorhergehenden Ansprüche zur dermalen Applikation, insbesondere für verletzte oder entzündete Haut.

24. Verwendung eines Schaumaerosols nach mindestens einem der vorhergehenden Ansprüche zur Applikation in Körperhöhlen.
